# EUROPEAN PATENT APPLICATION

(11) **EP 2 269 784 A1**
(43) Date of publication of application: **05.01.2011**
(21) Application number: 09164097.9
(22) Date of filing: 30.06.2009
(51) Int. Cl.: B26B 19/02, B26B 19/06, B26B 19/10, B26B 19/38, A45D 26/00

(54) **Personal care apparatus**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

A personal care apparatus (101) is presented, comprising a housing (117). This housing has a longitudinal axis (108) and is provided with a first end portion (102) and a second end portion (105). The first end portion (102) has a first end (103) having a first device configured for performing a function related to personal care (104). The second end portion (105) has a second end (106) having a second device configured for performing a function related to personal care (107). Said end portions (102, 105) are located in accordance with the longitudinal axis (108), and said ends (103, 106) are configured for pointing in different directions (114, 115). The first end portion (102) and the second end portion (105) are rotatable over an axis of rotation with respect to each other, which axis of rotation approximately corresponds to the longitudinal axis (108). This personal care apparatus allows the user to easily prevent the not in use device to have unintended interference with the user's skin or hairs. The personal care apparatus is very user friendly.

## Description

### FIELD OF THE INVENTION

A personal care apparatus comprising a housing having a longitudinal axis and provided with a first end portion and a second end portion, wherein the first end portion is provided with a first end having a first device configured for performing a function related to personal care and the second end portion is provided with a second end having a second device configured for performing a function related to personal care, which end portions are located in accordance with the longitudinal axis, wherein said ends are configured for pointing in different directions.

### BACKGROUND OF THE INVENTION

Personal care apparatuses having multiple functions are known from the art. For example, US 2007/0006463 A1 discloses an integrated shaving and precision trimming implement having a handle and both a trimming tool and a razor cartridge, each located at a different end of the handle. The trimmer implement of the device of US 2007/0006463 is, when not in use, stored in the handle of the implement. Storing the trimmer implement in the handle is regarded desirable in this document as the user cannot harm him or herself with the stored trimmer implement or inadvertently shorten hair which is not to be shortened with the stored trimmer implement, when using the razor cartridge. During a shaving operation in which the razor cartridge is use, the user desiring to switch to the use of the trimming tool, at that time stored in the handle, needs to undertake various actions to bring out this stored trimming tool. These various actions interrupt the hair shortening action and can be cumbersome and annoying.

### SUMMARY OF THE INVENTION

It is an object of the invention to provide a personal care apparatus having a first device configured for performing a function related to personal care and a second device configured for performing a function related to personal care, both devices available to the user for direct use, wherein the apparatus allows the user, while actively using one of said devices, to easily prevent unintentional interference with the user's skin and/or hairs by the other of said devices. Particularly, the object of the invention is to realize a user friendly personal care apparatus.

The object of the invention is realized by the personal care apparatus as defined in claim 1. Particularly, the personal care apparatus according to the invention has an axis of rotation over which the first end portion and the second end portion can be rotated with respect to each other, substantially corresponding to a longitudinal axis of the housing. The axis of rotation may coincide with the longitudinal axis or may run parallel or substantially parallel with the longitudinal axis.

A first device configured for performing a function related to personal care is located on a first end of a first end portion of the personal care apparatus, and a second device configured for performing a function related to personal care on a second end of a second end portion of the personal care apparatus. A device configured for performing a function related to personal care can have either or both a hair shortening function or a skin care function. Hair shortening functions in the context of this document encompass all functions to reduce the effective length of hairs. This not only includes shaving, cutting, trimming, clipping or the like, but also includes complete removal of the hairs by for example epilating, depilating or the application of light. Hair shortening can take place on any part of the body. The means provided to perform the hair shortening function can be specifically adapted to be applied to a certain part of the body, or can be for use on any hairy part of the body. Skin care functions can, for example, include cooling of the skin, dosing additives for e.g. improving skin properties, or to dose shaving soap, foam or the like. Other functions aimed at cosmetic product delivery, such as by means of ultrasound, ionophoresis, jet injection, microneedles, heat or massage, et cetera, or aimed at skin rejunevation, such as by means of light, radio frequencies, microdermabrasion, plasma or microcurrent, et cetera, or even acne treatment, such as by means of heat or light, are possible as well. As a group, these skin care functions are referred to as skin benefit functions. The places where a user might desire to shorten hairs can include the whole body, including the head, limbs, chest, armpits and pubic area. The user might desire to shorten hair on his or her own body, or on someone else's body. Similarly, the user might desire to apply skin care to his or her own body, or to someone else's body. For practical reasons, the discussion is limited to a user applying personal care to his or her own body. However, this is not to be interpreted as a scope limitation. It is very convenient for the user to have multiple devices configured for performing a function related to personal care offering different personal care functions available, integrated in one apparatus. While using such an apparatus, switching between said devices can take place without having to change between different apparatuses each offering only one of the desired functions. Not all body areas the user can desire to treat are easily reachable. This requires the user to manipulate his or her arm, wrist or hand, or a combination thereof, to reach the body area on which hairs are to be shortened. Sometimes this implies complex movements in which the personal care apparatus can limit the freedom of movement of the user. To prevent that while using one of said devices the user scratches, punches or otherwise discomforts himself or herself, or unintentionally shortens hair not to be shortened, with the second, at that time not to be used device, or more in general has unintended interference with said second device, the user can rotate the first end portion, provided with a first end having a first device configured for performing a function related to hair shortening, and the second end portion, provided a second end having a second device configured for performing a function related to personal care, with respect to each other along an axis substantially corresponding to the longitudinal axis of the apparatus. As the first end and the second end are configured for pointing in different directions, rotating the first and second end portions with respect to each other over an axis corresponding with the longitudinal axis of the apparatus will result in a change of the relative direction of the first and second ends. As the relative direction of the first and second ends changes, the relative direction, also to be understood as the relative orientation, of the first and second devices provided on these ends also changes due to this rotation. The user can thus easily adapt the personal care apparatus to prevent the unwanted shortening of hairs not to be shortened and to prevent being cut, scratched or otherwise discomforted by, or more in general prevent unintentional interference with the other, at that time not being in use device. Also, no action has to be taken to release a device from a closed or obstructed position as both devices are permanently available to the user, making the hair shortening operation efficient. All these factors contribute to the personal care apparatus of the invention being user friendly.

In a preferred embodiment of the personal care apparatus according to the invention the first device configured for performing a function related to personal care and the second device configured for performing a function related to personal care comprise hair shortening means. This allows the user to perform hair shortening operations requiring different hair shortening functions to be carried out without interruption for changing the apparatus. Such multi-function hair shortening operations can for example include both shaving and moustache trimming, or fine and gross trimming. Other combinations are possible as well. Not having to change between apparatuses during the hair shortening operation is very user friendly.

In a preferred embodiment of the personal care apparatus according to the invention, one of the first device configured for performing a function related to personal care and the second device configured for performing a function related to personal care comprises a hair shortening means and the other of said devices comprises a skin care means. In a practical embodiment the skin care means' function is related to the hair shortening means also provided with the apparatus. Examples of such related functions are epilating and cooling, razor blade shaving and provisioning of shaving soap, foam, gel or the like, and shaving and after shave care lotion provision. The combination of a hair shortening means and a skin care means allows the user to quickly change between both a hair shortening function and a function preventing, limiting or curing possible negative side effects of the hair shortening function. This is especially user friendly.

In a preferred embodiment of the personal care apparatus according to the invention, the first end portion and the second end portion are rotatable around an angle of 360 degrees with respect to each other. This feature allows the user to select any relative orientation of the first end and the second end possible and select the most suitable one for the hair shortening action to be performed. As any possible position can be selected, this is especially user friendly.

In a preferred embodiment of the personal care apparatus according to the invention, the first end portion and the second end portion are rotatable around an angle of 180 degrees with respect to each other. Such a rotation allows the user to select a position in which no unintended interference from the other of said devices will occur while at the same time the design and manufacturing costs can be limited as this requires a constructional less complex solution than full 360 degrees rotational freedom.

In a preferred embodiment of the personal care apparatus according to the invention the first end portion and the second end portion are continuously, i.e. stepless, rotatable with respect to each other. In this embodiment the user has the ultimate freedom to select any possible orientation of the first hair shortening means and the second hair shortening means as desired, available within the rotation allowed by the design of the personal care apparatus. As already mentioned the allowed rotation can be e.g. 360 or 180 degrees, but other allowed rotation angles are possible as well. The first end and the second end of the personal care apparatus, and thus the said first and second devices, can be positioned in any possible position with respect to each other over the allowed rotational angle. Such an embodiment is very user friendly.

In a preferred embodiment of the personal care apparatus according to the invention, the first end portion and the second end portion are stepwise rotatable with respect to each other. The rotation is thereby controlled in the sense that the rotation is indexed such that a designated number of releasably locked operational positions are available. This allows the user to secure the first end portion and the second end portion with respect to each other. When locked, the first end portion and the second end portion cannot move with respect to each other. Even if the user e.g. holds the apparatus at the second end portion while using the first device located at the first end of the first end portion, no undesired movement of the first end portion with respect to the second end portion can take place. This prevents unexpected behavior of the personal care apparatus, which unexpected behavior could lead to injury to the user and/or undesired hair shortening of hairs not to be shortened, and/or to hairs being shortened to a lesser degree than desired.

In a preferred embodiment of the personal care apparatus according to the invention, at least one of the first end portion and the second end portion has detents and at least the other one of the first end portion and the second end portion has notches for locking the first end portion and the second end portion with respect to each other. A detents and notch structure is particularly easy to design and manufacture when desiring to implement a step-wise rotational or releasably locking system.

In a preferred embodiment of the personal care apparatus according to the invention, that the housing has a curved longitudinal axis. Experiments have shown that a curved shape improves the ergonomics of the personal care apparatus. In case of a curved longitudinal axis, the axis of rotation roughly extends parallel to said curved longitudinal axis and may touch the longitudinal axis, may intersect the longitudinal axis or may run at some distance from the longitudinal axis.

In a preferred embodiment of the personal care apparatus according to the invention wherein the first device configured for performing a function related to personal care comprises a first hair shortening means, and the second device configured for performing a function related to personal care comprises a second hair shortening means, said first hair shortening means and said second hair shortening means having different hair shortening functions. A hair shortening function can for example be trimming, shaving or epilating. Such an embodiment is especially user friendly as it combines various different functions into one apparatus, instead that the user has to buy, store, transport, et cetera, two different apparatuses. Also the user experiences the user friendliness of the apparatus when needing to change between hair shortening functions, e.g. from trimming to shaving, without having to look for a second apparatus which also needs to be powered using an electric cord or have its batteries charged in order to continue hair shortening.

In a practical embodiment of the personal care apparatus according to the invention the first and second hair shortening means have different implements of the same hair shortening function, e.g. by having a wide and a small trimmer.

With reference to the claims it is noted that the invention also relates to all possible combinations of features and/or measures defined in the various claims.

It is to be noted that WO 2006/007874 A1 discloses an articulated razor having two shaving heads having different sizes for both standard purposes, such as shaving cheek and leg hair, and special purposes, such shaving the pubic area or moustache trimming. The first shaving head is mounted on a front end of the handle of the razor, the second shaving head is mounted on a support mounted on the back of the handle and which support is able to rotate with respect to the handle about an axis perpendicular to the handle axis. By rotating the support part of the second shaving head, the shaving head can be brought in either an open position, in which the shaving head projects outward of from the back end, or a closed position, in which the shaving head is retracted and lodged in the handle. During a shaving operation using the first shaving head, the user desiring to use the second shaving head needs to undertake various actions to bring out this second shaving head from its closed position, which interrupts the shaving action and can be cumbersome and annoying.

It is further to be noted that WO 2007/092291 A1 discloses a shaving device having a handle to which at a first end, a power trimmer is attached; and at a second end a razor cartridge. During operation of the power trimmer, its position relative to the razor cartridge is fixed. This means that during trimming actions, the user cannot control the orientation of the razor. A protective cap is provided to prevent the user getting injured from the not in use shaving means. Such a cap can easily get lost, exposing the user to the risk of being scratched or otherwise injured by the unprotected shaving means, or severing hairs which should not be reduced in length. This is especially true during ergonomically complex trimming actions.

It is also to be noted that European patent application EP 1938931 A2 discloses a hair trimmer with a rotatable detented head. This trimmer has a single hair trimming means housed in a blade assembly housing at one end of the trimmer handle. The blade assembly housing can rotate with respect to the handle along an axis of rotation corresponding to a longitudinal axis of the handle. The device according to this patent application has one hair trimming means only. Consequently, a user needing to use more than one hair trimming means must employ different trimmers which is costly, requires extra storage capacity and is potentially confusing.

### BRIEF DESCRIPTION OF THE DRAWINGS

A detailed description of the invention is provided below. The description is provided by way of a non-limiting example to be read with reference to the drawings in which:
Figure 1A shows a first schematic side view of a first embodiment of the personal care apparatus according to the invention according to a first scale.
Figure 1B shows a second schematic side view of the first embodiment of the personal care apparatus according to the invention according to a second scale.
Figure 1C shows the first embodiment of the personal care apparatus during a hair shortening operation according to a third scale.
Figure 2 shows a schematic side view of a second embodiment of the personal care apparatus according to the invention.

### DETAILED DESCRIPTION OF THE EMBODIMENTS

In figures showing the same embodiment or the same parts thereof, the same numbers are used for the same parts.

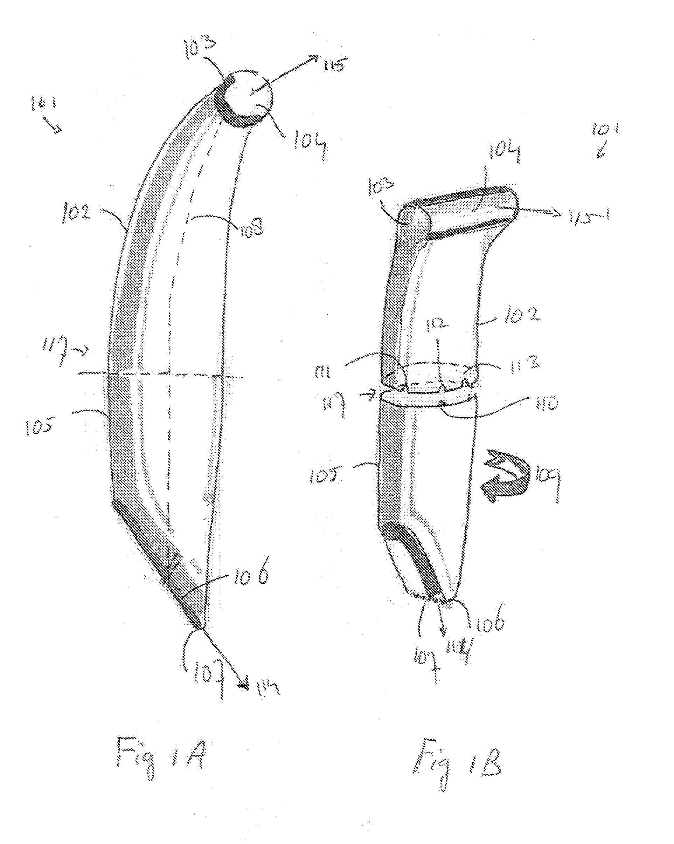

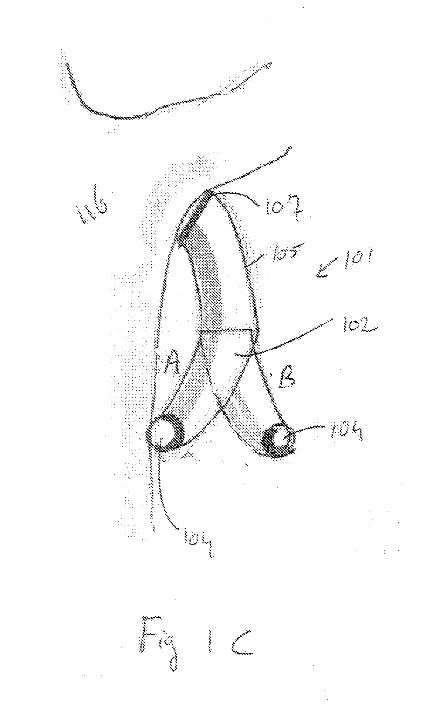

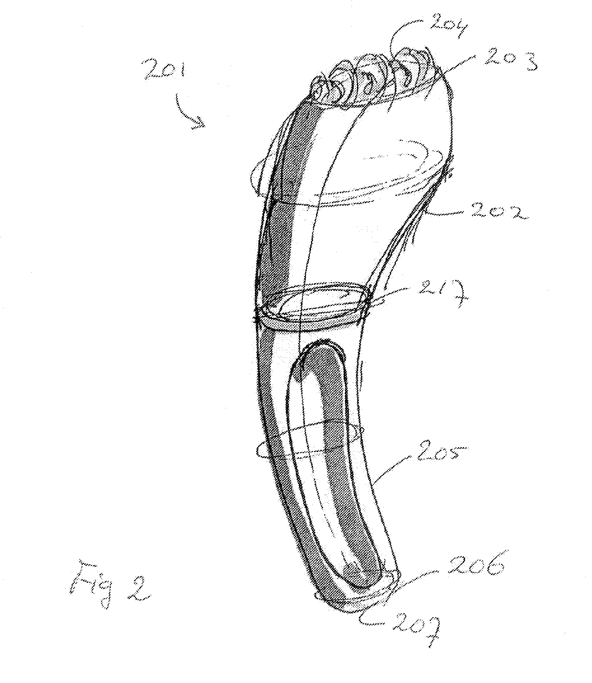

Figures 1A, 1B and 1C show the same embodiment of a personal care apparatus according to the invention. A personal care apparatus 101 is shown which has a first end portion 102 having a first end 103 which has a first device configured for performing a function related to personal care 104. This first device configured for performing a function related to personal care 104 is in this embodiment a foil shaving element. Foil shaving elements are known from the art, e.g. from US patent US 4928389, and not discussed any further. The first device configured for performing a function related to personal care 104 points in a direction 115. The personal care apparatus 101 further has a second end portion 105 which has a second end 106 comprising a second device configured for performing a function related to personal care 107. This second device configured for performing a function related to personal care 107 is in this embodiment a hair trimming element. Hair trimming elements are known from the art, e.g. from US patent US 4610085, and not discussed any further. The second device configured for performing a function related to personal care points in direction 114. In other embodiments, the first device configured for performing a function related to personal care and the second device configured for performing a function related to personal care can have different functions compared to the functions of the embodiment of figures 1A and 1B. In some embodiments, for example the embodiment shown in figure 2, one of said devices can be a so-called skin care means. In some other embodiments of the personal care apparatus according to the invention both of said devices can be so-called skin care means. Figure 1A shows a longitudinal axis 108 over which the first end portion 102 and the second end portion 105 can rotate with respect to each other. This rotation is indicated in figure 1B by arrow 109. For illustration purposes, the first end portion 102 and the second end portion 105 are shown detached in figure 1B. During operation the end portions 102 and 105 are attached like shown in figure 1A. Again referring to figure 1B, a detent 110 and three notches 111, 112 and 113 are visible.

The personal care apparatus 101 is provided with a driving means, e.g. an electric motor (not shown), which can either be powered by power means (not shown) such as a battery, rechargeable battery or mains power using a cord. The driving means serves for driving said first and second devices. The driving means can be controlled using a switch (not shown). This switch can be a plain on/off switch or in other embodiments more complex switches, e.g. able to control the amount of power delivered to one or both of the said devices, can be provided. In other embodiments, one or two of said devices might not need to be powered. In embodiments where none of the devices requires power, the driving means, power means and switch can be absent.

The user can rotate the first end portion 102 and the second end portion 105 with respect to each other over the longitudinal axis 108 in order to optimize the ergonomics of the personal care apparatus 101 to fit the needs of the shaving operation at hand. Rotating the two end portions 102 and 105 also effects the relative orientation of the two devices 104 and 107. This can be seen by comparing the relative orientations of directions 114 and 115 in figure 1A and the relative orientations of directions 114' and 115' in figure 1B. As the relative orientations of the two devices are changed, the user has an opportunity to easily adapt the personal care apparatus to 101 prevent the unwanted shortening of hairs not to be shortened and to prevent being cut, scratched or otherwise discomforted by the not to be used device. This is illustrated in Figure 1C which shows the personal care apparatus 101 being used to shorten armpit hairs of a user 116 using the second device 107. Figure 1C shows two relative orientations of the two end portions 102 and 105, viz. an orientation A in which the first device configured for performing a function related to personal care 104 touches the user 116, and an orientation B in which the first device configured for performing a function related to personal care 104 does not touch the user 116. For the sake of clarity the user's hand holding the personal care apparatus 101 is not shown in figure 1C.

Now specifically referring to figure 1B, a detent 110 and three notches 111, 112 and 113 are shown. In the relative orientation shown in figure 1B, the detent 110 interacts with notch 112, forming a releasable locking structure. When the first and second end portions 102 and 105 are rotated, detent 110 can releasably lock with e.g. the notch 111 or 113. In other embodiments more notches and/or detents or a totally different releasable locking mechanism can be provided. Such a locking mechanism can be known per se. Also, embodiments without specific locking or arresting mechanism are feasible. In such embodiments the two end portions can for example be held in their relative position by the friction of the rotational coupling of the two end portions.

Figure 2 shows an embodiment of the personal care apparatus according to the invention in which the second device configured for performing a function related to personal care is a skin care means. A personal care apparatus 201 is shown which has a first end portion 202 having a first end 203 which has a first device configured for performing a function related to personal care 204. This first device configured for performing a function related to personal care 204 is in this embodiment an epilating element. Epilating elements are known from the art, e.g. from US patent US 4279253, and not discussed any further. The personal care apparatus 201 further has a second end portion 205 which has a second end 206 comprising a second device configured for performing a function related to personal care 207. This second device configured for performing a function related to personal care 207 is in this embodiment a cooling element. Such a cooling element can e.g. be realized by cooling a surface using a so-called Peltier device. It is known from the art that users of an epilator often experience pain during epilating. A common cure for this pain is application of ice, e.g. in the form of ice cubes after epilating. Having an apparatus combining an epilator and a cooling element is very user friendly as the user only has to switch from one side, viz. the first end 203, to the other side, viz, the second end 206, to start curing his or her painful skin. Although applying cold to ones skin directly after epilating can be comforting, doing so unintentionally is experienced as unpleasant by most users. By rotating the first end portion 202 with respect to the second end portion 205 unwanted contact between the cooling element and the user's skin can be prevented similar as illustrated in figure 1C.

While the invention has been illustrated and described in detail in the drawings and in the foregoing description, the illustrations and the description are to be considered illustrative or exemplary and not restrictive. The invention is not limited to the disclosed embodiments. It is noted that the personal care apparatus according to the invention and all its components can be made by applying processes and materials known per se. In the set of claims and the description the word "comprising" does not exclude other elements and the indefinite article "a" or "an" does not exclude a plurality. Any reference signs in the claims should not be construed as limiting the scope. It is further noted that all possible combinations of features as defined in the set of claims are part of the invention.

## Claims

1. A personal care apparatus (101, 201) comprising:
a housing (117, 217) having a longitudinal axis (108) and provided with a first end portion (102, 202) and a second end portion (105, 205),
wherein the first end portion (102, 202) is provided with a first end (103, 203) having a first device configured for performing a function related to personal care (104, 204) and the second end portion (105, 205) is provided with a second end (106, 206) having a second device configured for performing a function related to personal care (107, 207),
which end portions (102, 202, 105, 205) are located in accordance with the longitudinal axis (108), wherein said ends (103, 203, 106, 206) are configured for pointing in different directions (114, 115, 114', 115'),
wherein the first end portion (102, 202) and the second end portion (105, 205) are rotatable over an axis of rotation with respect to each other, which axis of rotation substantially corresponds to the longitudinal axis (108).

2. The personal care apparatus of claim 1, **characterized in that** the first device configured for performing a function related to personal care (104, 204) and the second device configured for performing a function related to personal care (107, 207) comprise hair shortening means.

3. The personal care apparatus of claim 1, **characterized in that** one of the first device configured for performing a function related to personal care (104, 204) and the second device configured for performing a function related to personal care (107, 207) comprises a hair shortening means and the other of said devices comprises a skin care means.

4. The personal care apparatus according to claim 1, 2 or 3, **characterized in that** the first end portion (102, 202) and the second end portion (105, 205) are rotatable around the axis of rotation over an angle of 360 degrees with respect to each other.

5. The personal care apparatus according to claim 1, 2, or 3, **characterized in that** the first end portion (102, 202) and the second end portion (105, 205) are rotatable around the axis of rotation over an angle of 180 degrees with respect to each other.

6. The personal care apparatus according to any one of the preceding claims, **characterized in that** the first end portion (102, 202) and the second end portion (105, 205) are continuously rotatable with respect to each other.

7. The personal care apparatus according to any one of the preceding claims, **characterized in that** the first end portion (102, 202) and the second end portion (105, 205) are stepwise rotatable with respect to each other.

8. The personal care apparatus according to claim 7, **characterized in that** at least one of the first end portion (102, 202) and the second end portion (105, 205) has detents (110) and at least the other one of the first end portion (102, 202) and the second end portion (105, 205) has notches (111,112, 113) for locking the first end portion (102, 202) and the second end portion (105, 205) with respect to each other.

9. The personal care apparatus according to any one of the preceding claims, **characterized in that** the housing (117, 217) has a curved longitudinal axis (108).

10. The personal care apparatus according to claim 2 or any of the claims 4 to 9 insofar referring to claim 2, **characterized in that** the first device configured for performing a function related to personal care (104), and the second device configured for performing a function related to personal care (107), have different hair shortening functions.
